# EUROPÄISCHE PATENTSCHRIFT

(11) **EP 3 592 331 B1**
(45) Veröffentlichungstag und Bekanntmachung des Hinweises auf die Patenterteilung: **30.12.2020**
(21) Anmeldenummer: 18707736.7
(22) Anmeldetag: 06.03.2018
(51) Int. Cl.: A61K 9/00, A61K 49/04

(54) **KONTRASTMITTEL FÜR MIKROANGIOGRAFIE**
CONTRAST MEDIUM FOR MICROANGIOGRAPHY
AGENT DE CONSTRASTE POUR MICRO-ANGIOGRAPHIE

(30) Priorität: 09.03.2017 CH 2842017
(43) Veröffentlichungstag der Anmeldung: 15.01.2020
(73) Patentinhaber: Pitengo GmbH, 6215 Beromünster (CH)
(72) Erfinder: STEGER, Beat, 6749 Sobrio (CH); HLUSHCHUK, Ruslan, 3018 Bern (CH); DJONOV, Valentin, 3012 Bern (CH)
(74) Vertreter: Pestalozzi, Deborah
(86) Internationale Anmeldenummer: PCT/EP2018/055463
(87) Internationale Veröffentlichungsnummer: WO 2018/162473

(56) Entgegenhaltungen:
- WO-A2-2008/034270
- WO-A2-2009/081169
- ZAGORCHEV LYUBOMIR ET AL: "Micro computed tomography for vascular exploration", JOURNAL OF ANGIOGENESIS RESEARCH, BIOMED CENTRAL LTD, Bd. 2, Nr. 1, 5. März 2010 (2010-03-05), Seite 7, XP021070689, ISSN: 2040-2384
- GRABHERR S ET AL: "POSTMORTEM ANGIOGRAPHY: REVIEW OF FORMER AND CURRENT METHODS", AMERICAN JOURNAL OF ROENTGENO, AMERICAN ROENTGEN RAY SOCIETY, US, Bd. 188, Nr. 3, 1. März 2007 (2007-03-01), Seiten 832-838, XP008078071, ISSN: 0361-803X, DOI: 10.2214/AJR.06.0787

## Beschreibung

### TECHNISCHES GEBIET

Die vorliegende Erfindung betrifft ein Kontrastmittel für postmortale Mikroangiografie, d.h. zur digitalen Bildgebung eines Gefässsystems, insbesondere von Kleintieren, wie beispielsweise einer Maus, einer Ratte, oder anderer Labortiere, sowie einzelner tierischer und menschlicher Organe mittels eines röntgenbasierten Bildgebungsverfahrens, insbesondere eines Nano- oder Micro-CT-Geräts.

### STAND DER TECHNIK

Ziel der Mikroangiografie ist es, kleinste Gefässe, d.h. Kapillaren einzelner Organe oder ganzer Körper dreidimensional zu visualisieren und zwecks Analyse exakt wiederzugeben. Vor allem für die pharmakologische Forschung, aber auch in der Forensik, ist eine Analyse der intakten Gefässe erforderlich. Bisher wurden im Wesentlichen zwei Technologien zur Darstellung der Mikrogefässe verwendet: einerseits die sogenannte Ausguss-Methode, und zweitens die radiologische Kontrastmitteldarstellung, insbesondere die Microfil®-Methode.

3D-Darstellungsmethoden wie Micro Computed Tomography (Micro-CT) haben in den vergangenen Jahren vermehrte Aufmerksamkeit erregt. Die Darstellung des Gefässsystems erfordert eine Perfusion mit einem röntgenopaken (radiopaquen) Kontrastmittel zur Visualisierung mittels Micro-CT. In der Gefässforschung wurde Micro-CT bisher in Kombination mit verschiedenen Kontrastmitteln verwendet, um das Gefässsystem verschiedener Organe, wie z.B. Hirn, Herz, Leber, Niere, Lungen, sowie der hinteren Extremitäten, als auch von Tumoren darzustellen. Diese Studien waren aber stets hinsichtlich der Auflösung limitiert, oder aber durch eine unvollständige Füllung und fehlerhafte Perfusion (auch aufgrund der relativ hohen Viskosität des verwendeten Kontrastmittels) (z.B. Perrien, D.S., 2016).

Bei der Ausguss-Methode (engl.: vascular corrosion casting) wird Giessmaterial, z.B. ein zu polymerisierendes, auf Polyurethan basiertes Mittel, in die Gefässe injiziert (z.B. Meyer et al., 2007 & 2008; Krucker et al., 2006). Anschliessend, nach dessen Polymerisation, wird das umliegende Gewebe chemisch mazeriert und verdaut. Die so entstandenen Gefässausgüsse werden anschliessend mittels Rasterelektronenmikroskopie (REM) oder radiologisch dreidimensional ausgewertet. Es besteht auch die Möglichkeit, ein dreidimensionales Gefässmodell zu erstellen. Der schwerwiegende Hauptnachteil dieser Methode entsteht durch die Gewebemazeration, da diese eine (anschliessende) histologische Untersuchung ausschliesst und dementsprechend auch nicht einmal eine Lokalisierung innerhalb des Gewebes möglich ist. Ein weiterer Nachteil dieser Methode beim Einsatz der Rasterelektronmikroskopie ist die limitierte Darstellbarkeit von Gefässen, da man zwar ein Bild von Gefässen an der Aussenoberfläche des Gefässausgusses erhält, aber nicht im Inneren des Ausgusses.

Die Microfil®-Methode eignet sich vor allem für die Darstellung von Gefässen mit einem Durchmesser von bis zu 100 µm. Da dieser Gefässdurchmesser kaum Rückschlüsse auf das Kapillargebiet zulässt, wird die Anwendung vom Benutzer auf verschiedene Arten modifiziert. Das heisst, der Benutzer variiert je nach Bedarf jeweils die Zusammensetzung der Kontrastmittelsubstanz durch Verdünnung oder anderweitige Veränderung, sodass das Kontrastmittel in kleinere Gefässe eindringen kann. In der Regel kann so ein Kapillargebiet von bis zu 10 µm erreicht und dargestellt werden. Die Art der Verdünnung oder Veränderung der Kontrastmittelzusammensetzung erfolgt jedoch nach keinem bekannten Standard, sondern individuell. Die Applikation erfolgt in der Regel manuell. Gemäss publizierten Studien ist die Perfusion, wahrscheinlich infolge einer relativ hohen Viskosität, trotzdem mangelhaft (Perrien, D.S. 2016).

Der Austausch von Sauerstoff und Metaboliten findet hauptsächlich auf Höhe des Kapillarbettes statt, also bei Gefässdurchmessern von ca. 4 bis 10 µm. Präklinische Studien beschränken sich in der Regel auf die Darstellung von Mikrogefässen mittleren Durchmessers (ca. 15 µm). Mittlerweile stehen der Forschung MikroCT-Scanner bzw. Mikro-CT-Geräte zur Verfügung, welche Kapillaren bis zu einem Durchmesser von 3-4 µm darstellen können, sofern diese Kapillaren mit einem geeigneten zugefügten Kontrastmittel auch erreicht und gefüllt werden können.

Entsprechend besteht ein grosser Bedarf für ein Kontrastmittel, welches genügend weit in die kleinsten Gefässe vordringen kann und eine möglichst Artefakt-freie Visualisierung der Microvaskulatur bzw. eine morphometrische 3D-Bildanalyse mittels Micro-CT ermöglicht (siehe auch Zagorchev et al., 2010).

Die internationale Anmeldung WO2008/034270 enthüllt Kontrastmittel für die diagnostische Mikroangiographie mit guter Kapillareindringung.

WO2009/081169 enthüllt Kontrastmittel, die unter anderem auch iodiertes Öl umfassen.

### DARSTELLUNG DER ERFINDUNG

Entsprechend löst die vorliegende Erfindung die Aufgabe, ein verbessertes Kontrastmittel zur Verfügung zu stellen, welches bis in Kapillaren mit einem Durchmesser von bis zu 3-4 µm einzudringen vermag und so eine Darstellung der Gefässe mittels Micro-CT-Verfahrens ermöglicht. Ebenso besteht ein grosser Bedarf für ein verbessertes *ex vivo* Angiografieverfahren, welches eine reproduzierbare Darstellbarkeit des Gefässsystems erlaubt. Mit der Entwicklung der erfindungsgemässen Kontrastlösung steht nun ein solches verbessertes Kontrastmittel, welches die Nachteile des momentanen Standes der Technik überwindet, sowie ein verbessertes Angiografieverfahren zur Verfügung.

Die oben beschriebene Aufgabe wird durch das Kontrastmittel gemäss Anspruch 1 gelöst, bzw. durch den in Anspruch 10 beanspruchten Kit-of-Parts. Die Bereitstellung des verbesserten Kontrastmittels erfolgt vorzugsweise nach dem Herstellungsverfahren gemäss Anspruch 14, und das *ex vivo* Mikroangiografie-Verfahren gemäss Anspruch 15 ermöglicht eine reproduzierbare und damit optimierte Anwendung des erfindungsgemässen Kontrastmittels.

Das erfindungsgemässe Kontrastmittel, welches es ermöglicht, Kapillaren mit einem Durchmesser von bis zu 3-4 µm darzustellen, wird *ex vivo* eingesetzt. Das erfindungsgemässe Kontrastmittel für *ex vivo* bzw. postmortale Mikroangiografie dient vorzugsweise zur digitalen Bildgebung und somit der Untersuchung des Gefässsystems einer Maus oder einer Ratte, oder anderer Labortiere und menschlicher Organe mittels eines Micro-CT-Geräts. Das erfindungsgemässe Kontrastmittel enthält ein iodiertes, verestertes Öl, vorzugsweise ein iodiertes, verestertes Leinsamenöl (Leinöl) oder ein iodiertes, verestertes Mohnsamenöl (Mohnöl). Zudem weist das erfindungsgemässe Kontrastmittel ein Polyurethan und einen Härter auf, sowie ein Keton als Lösungsmittel. Das Keton ist vorzugsweise ausgewählt aus der folgenden Gruppe: Butanon (bzw. 2-Butanon bzw. Methylethylketon bzw. C₄H₈O), Aceton (bzw. 2-Propanon bzw. Dimethylketon bzw. C₃H₆O), oder 3-Pentanon (bzw. Diethylketon bzw. C₅H₁₀O). Dabei wird als Lösungsmittel insbesondere 2-Butanon oder Aceton bevorzugt, am meisten bevorzugt ist 2-Butanon. Alternativ kann unter Umständen als Lösungsmittel auch Methylenchlorid verwendet werden. Die Mischung von dem iodierten, veresterten Öl mit dem Keton wird für den Zweck dieser Anmeldung als "Kontrastlösung" bezeichnet.

Eine besonders bevorzugte Ausführungsform des Kontrastmittels enthält ausserdem einen Farbstoff, wobei der Farbstoff vorzugsweise ein blauer Farbstoff ist (z.B. BlueDye von VasQtec). Im Falle der Beifügung eines Farbstoffs, ist dieser auch Teil der zum Zweck dieser Anmeldung als "Kontrastlösung" bezeichneten Mischung.

Eine bevorzugte Ausführungsform enthält, wie oben erwähnt, als iodiertes verestertes Öl ein iodiertes, verestertes Leinsamenöl. Das erfindungsgemässe Kontrastmittel ist somit eine jodhaltige und vorzugsweise auch farbstoffhaltige, polymerisierende Substanz, welche vorzugsweise auf iodiertem, verestertem Leinsamenöl basiert. Vorzugsweise ist das iodierte, veresterte Leinsamenöl Ethyl-9,12,15-triiodo-octadecatrienoat, bzw. Ethyl-Linolenat.

Das erfindungsgemässe Kontrastmittel verfügt vorzugsweise über autofluoreszierende Eigenschaften und führt dazu, dass sich in der initialen Applikationsphase die Gefässe vorzugsweise blau verfärben, was die optische Kontrolle der Injektion erleichtert.

Vor der Applikation bzw. Injektion in den zu untersuchenden Körper bzw. selektiv in das zu untersuchende Organ wird die Kontrastlösung nach einem definierten Schema mit dem erwähnten Polyurethan (PU-) Resin und mit einem Härter gemischt.

Das zur Herstellung des erfindungsgemässen Kontrastmittels verwendete Polyurethan ist vorzugsweise ein Polyisocyanat-Präpolymer. Es handelt sich dabei vorzugsweise um ein aliphatisches Isocyanat. Vorzugsweise weist das Isocyanat eine aromatische oder vorzugsweise eine aliphatische Gruppe auf. Damit das Polyurethan flexibel bleibt, werden normalerweise Polyether eingesetzt. Es ist besonders vorteilhaft, wenn das Polyurethan einen Polyester, oder vorzugsweise einen Polyether enthält, insbesondere bevorzugt einen Polyether, der aus Ethylenglykol- und/oder Propylenglykol-Resten gebildet ist. Vorzugsweise weist das Polyurethan einen Kettenverlängerer auf, insbesondere ein Diol oder vorzugsweise einen Diamin-basierten Kettenverlängerer, insbesondere bevorzugt Diethylmethylbenzoldiamin. Eine besonders für die Mikroangiografie geeignetes Kontrastmittel weist als Polyurethan 4,4'-Methylendi(cyclohexyl-isocyanat) (HDMI) auf, bzw. 4,4'-Dicyclohexylmethan-diisocyanat.

Der im erfindungsgemässen Kontrastmittel verwendete Härter, welcher vorzugsweise erst kurz vor der Einspritzung in den Körper, bzw. selektiv in ein Organ, der Mischung aus dem iodierten, veresterten Öl, Butanon, und PU beigemischt wird, ist vorzugsweise ein modifiziertes aromatisches Diamin, insbesondere bevorzugt ein Diethylmethylbenzoldiamin, beispielsweise 2,6-diamino-3,5-diethyltoluen. Ein für die Verwendung im erfindungsgemässen Kontrastmittel besonders vorteilhafter Härter weist eine Mischung von zwei Isomeren von Diethylmethylbenzoldiamin auf, am meisten bevorzugt ein Isomerengemisch von 2,6-Diamino-3,5-diethyltoluol und 2,4-Diamino-3,6-diethyltoluol im Verhältnis 7:3.

Vorteilhafterweise ist das iodierte, veresterte Öl zu 20-60%, vorzugsweise zu 22-45%, insbesondere bevorzugt zu 24-30% im Kontrastmittel enthalten (jeweils Volumenprozent). Das Keton, bzw. vorzugsweise das 2-Butanon, ist vorteilhafterweise zu 7-30%, vorzugsweise zu 10-25%, insbesondere bevorzugt zu 14-22% im Kontrastmittel enthalten (jeweils Volumenprozent).

Das Polyurethan ist vorteilhafterweise zu 25-60%, vorzugsweise zu 35-50%, insbesondere bevorzugt zu 38-50%, und am meisten bevorzugt zu 43-47% im Kontrastmittel enthalten (jeweils Volumenprozent).

Der Härter ist vorteilhafterweise zu 4-10%, vorzugsweise zu 5-9%, insbesondere bevorzugt zu 6-8% im Kontrastmittel enthalten (jeweils Volumenprozent).

Die Erfindung betrifft ausserdem einen Kit-of-Parts für Mikroangiografie, umfassend:
- einen ersten Behälter, der das oben erwähnte iodierte, veresterte Öl, und das erwähnte Keton, bzw. vorzugsweise das 2-Butanon enthält; und
- einen zweiten Behälter, der das oben erwähnte Polyurethan enthält; und
- einen dritten Behälter, der den oben erwähnten Härter enthält.

Der erste Behälter enthält dabei vorzugsweise eine erste Mischung aus 2-4 ml, vorzugsweise 2.5-2.8 ml des iodierten, veresterten Öls, vorzugsweise des iodierten, veresterten Leinsamenöls, und 2-3 ml, vorzugsweise 2.2-2.9 ml des Ketons, bzw. des 2-Butanons. Das heisst, der erste Behälter enthält die "Kontrastlösung". Dabei enthält der erste Behälter vorzugsweise zusätzlich den oben erwähnten Farbstoff, vorzugsweise einen blauen Farbstoff, welcher im Falle seiner Beimischung auch zu der "Kontrastlösung" gehört. Die Beigabe einer Messerspitze des Farbstoffs, was ca. 0.2 g des Farbstoffs entspricht, reicht bereits für die vorliegende Anwendung. Der zweite Behälter enthält vorzugsweise 4-7 ml, insbesondere bevorzugt 4.5-5 ml des Polyurethans; und der dritte Behälter enthält vorzugsweise 0.5-1.5 ml, insbesondere bevorzugt 0.8-1.2 ml des Härters. Vorzugsweise liegt in dem zu injizierenden Gemisch das Volumenverhältnis des Polyurethans zum Härter im Bereich von 100:10 bis 100:25, insbesondere bevorzugt im Bereich von 100:16 bis 100:19.

Vorzugsweise enthält der Kit-of-Parts des Weiteren
- eine erste Spritze zur Aufnahme des Inhalts des ersten Behälters und des zweiten Behälters, vorzugsweise eine Spritze mit 12 ml Volumen;
- eine zweite Spritze zur Aufnahme des Inhalts des dritten Behälters, vorzugsweise eine Spritze mit 1 ml Volumen;
- einen Mischbehälter zur Vermengung des Inhalts der ersten Spritze und der zweiten Spritze;
- einen Dispenser zur Steuerung der ersten Spritze und der zweiten Spritze, wobei der Dispenser eine Vorrichtung zur Aufnahme eines jeweils ersten Endes der ersten und der zweiten Spritze aufweist;
- und vorzugsweise ein Adapterelement zur Aufnahme eines jeweils zweiten Endes der ersten und der zweiten Spritze und zur Aufnahme eines ersten Endes des Mischbehälters.

Die Erfindung betrifft ausserdem ein Verfahren zur Herstellung des oben beschriebenen Kontrastmittels für Mikroangiografie, zur digitalen Bildgebung eines Gefässsystems einer Maus oder einer Ratte mittels eines Micro-CT- (µCT-) Geräts, wobei das Herstellungsverfahren die folgenden Schritte aufweist:
a) Bereitstellung einer ersten Mischung von iodiertem, verestertem Öl, vorzugsweise von iodiertem, verestertem Leinsamenöl, mit einem Keton, vorzugsweise mit 2-Butanon, in einem ersten Behälter;
b) Bereitstellung eines Polyurethans in einem zweiten Behälter;
c) Bereitstellung eines Härters in einem dritten Behälter;
d) Vermengung und Mischen des Inhalts des ersten Behälters mit dem Inhalt des zweiten Behälters zu einer zweiten Mischung;
e) Vermengung des Inhalts des dritten Behälters mit der zweiten Mischung von Schritt d) in einem Mischelement unmittelbar vor der Einspritzung in das Gefässsystem der Maus oder der Ratte; wobei vorzugsweise ein Mischverhältnis von 100:16 des Polyurethans zum Härter verwendet wird.

Die Erfindung betrifft des Weiteren ein Verfahren für Mikroangiografie zur digitalen Bildgebung eines Gefässsystems eines Tierkörpers, insbesondere einer Maus oder einer Ratte, mittels eines Micro-CT-Geräts, aufweisend die folgenden Schritte:
- Bereitstellung des oben beschriebenen Kontrastmittels, vorzugsweise nach dem oben beschriebenen Verfahren;
- Kanülierung und Ausspülung bzw. Ausbluten des zu untersuchenden Tierkörpers, vorzugsweise mit einer Klarlösung, insbesondere mit PBS (phosphatgepufferte Salzlösung, engl.: phosphate buffered saline), wobei vorzugsweise für eine Maus eine Spülmenge von 20-100 ml, und alternativ für eine Ratte eine Spülmenge von 20-200 ml verwendet wird;
- Injektion des Kontrastmittels, vorzugsweise mit gleichmässiger Flussrate und vorzugsweise bei möglichst gleichmässigem Druck, wobei die Flussrate vorzugsweise maximal 3 ml/min, insbesondere bevorzugt maximal 1.5 ml/min beträgt.

Die Applikation bzw. Injektion des Kontrastmittels kann entweder manuell mittels eines Dispensers erfolgen, oder alternativ mittels einer Injektionspumpe bzw. mittels eines vorzugsweise modifizierten, bzw. einen auf individuelle Anforderungen angepassten Perfusors, mittels welchem ein bestimmtes Volumen pro Zeiteinheit eingehalten werden kann.

Für eine Maus werden normalerweise zwischen 1-12 ml des Kontrastmittels injiziert, und für eine Ratte normalerweise zwischen 1-30 ml des Kontrastmittels, abhängig vom zu untersuchenden Zielorgan.

Die Injektion des Kontrastmittels in die Maus oder Ratte erfolgt vorzugsweise während eines Zeitfensters von 1-6 min, wobei vorzugsweise für eine Maus oder eine Ratte eine Einspritzrate von 0.5-12 ml/min, insbesondere bevorzugt von 1-3 ml/min verwendet wird, am meisten bevorzugt von maximal 1.5 ml/min.

Nach der Injektion des Kontrastmittels wird vorzugsweise eine Aushärtung des Kontrastmittels im Tierkörper abgewartet. Als Nächstes wird das betreffende Organ oder der betreffende Körperteil ausgeschnitten und chemisch fixiert, und anschliessend mittels eines Micro-CT-Geräts gescannt.

Das erfindungsgemässe Kontrastmittel eignet sich in erster Linie für Versuchszwecke, d.h. in der präklinischen Forschung für die Perfusion von Kleintierkörpern, insbesondere von Maus und Ratte. Es kann aber beispielsweise auch für die selektive Perfusion einzelner Gebiete oder Organe von grösseren Versuchstieren verwendet werden, so z.B. von Kaninchen, Hunden, Fischen, Schafen, Minischweinen, etc. Diese werden beispielsweise in der orthopädischen oder zahnmedizinischen Forschung (Zahnersatz, Knochenersatz, etc.) eingesetzt. Zu diesem Zweck wird das Kontrastmittel spezifisch in jene Arterie, deren "Endgebiet" dargestellt werden soll, eingespritzt. Auf diese Weise kann in einzelne Organe oder Körperteile, wie z.B. in den Unterkiefer, etc. selektiv Kontrastmittel injiziert werden und die entsprechenden Organe oder Körperteile können dann dargestellt werden. Das erfindungsgemässe Kontrastmittel eignet sich auch für die Anwendung in der Forensik, und dabei insbesondere auch bei der gerichtsmedizinischen Untersuchung von menschlichen Leichen.

Die Bereitstellung des erfindungsgemässen Kontrastmittels öffnet neue Anwendungsgebiete für *ex vivo* Micro-CT Technologie in diversen Gebieten der Biomedizinischen Forschung. Während bei der PU-Verdauungsmethode nach dem Stand der Technik nach dem CT-Scan umliegendes Gewebe weg-verdaut werden muss, um ein 3D-Modell des Gefässsystems zu erhalten, erlaubt es das erfmdungsgemässe, nichtdestruktive Verfahren, einerseits hochauflösende Bilder des Gefässsystems zu erhalten und andererseits können anschliessend bereits gescannte Proben zudem als Grundlage für histologische oder elektronenmikroskopische Untersuchungen dienen, da umliegendes Gewebe intakt bleibt. Ebenso können die besonders interessierenden Organteile rausgeschnitten und bei einer noch höheren Auflösung gescannt werden. Die Bildanalyse erfolgt dann unter Verwendung einer geeigneten Quantifizierungs-Software.

Nachgewiesen wurde der vorteilhafte Einsatz des Kontrastmittels beispielsweise für die Morphometrie des Nieren-Gefässsystems, einschliesslich einer Quantifizierung von Nieren-Glomeruli bei Mäusen mit einer Auflösung von bis unter 2.5 Micrometer (voxel side size) (Shokiche, C.C. et al, 2016), sowie in der korrelativen Darstellung des Gefässsystems und Muskelgewebes der hinteren Extremität der Maus (Schaad et al., 2017). Bei einer Perfusion des Tierkörpers mit Microfil® (Fluitec), dem bisherigen Standard-Bildgebungsverfahren, kann normalerweise eine Auflösung von bis zu ca. 50-100 Micrometer erreicht werden. Um diese Auflösung zu verbessern, wurde das Microfil® von Angiografie-Technikern individuell verdünnt, was je nach Verdünnungsgrad eine Auflösung von bis zu ca. 12 Micrometern erlaubte. Die Perfusion von kleineren Gefässen und Kapillaren ist aber infolge mehrerer Faktoren, wie beispielsweise der höheren Viskosität, mangelhaft (Perrien D.S., 2016).

Das erfindungsgemässe Kontrastmittel mit der Kontrastlösung hingegen dringt bis in die kleinsten Kapillaren von bis zu 3-4 Mikrometern Durchmesser ein und erlaubt so eine detailliertere Darstellung des Gefässsystems. Das erfindungsgemässe Anwendungsverfahren bietet auch eine reproduzierbare niedrige Viskosität (im Vergleich zu den diversen individuellen Modifikationen der Microfil®-Methode).

Ein weiterer Vorteil des erfindungsgemässen Kontrastmittels ist der, dass die Polymerisierung dem Testobjekt zusätzliche Stabilität verleiht, was insbesondere während des Micro-CT-Scans von Vorteil ist, da es die Qualität der Bildgebung verbessert. Ebenso weist das neue auf der Kontrastlösung basierende Kontrastmittel einen hohen Röntgen-Absorptionsgrad auf, welcher nahe bei demjenigen von Knochengewebe ist. Dies vereinfacht eine auf Schwellenwerten basierende Segmentierung der darzustellenden Gefässe und t deren Visualisierung.

Die Aushärtungs- und Autofluoreszenz-Eigenschaften des erfindungsgemässen Kontrastmittels erlauben somit zusammenfassend eine korrelative Herangehensweise, d.h. nach der Micro-CT-Bildgebung und Definition der weiter zu erforschenden Gewebeabschnitte, kann zusätzlich eine morphologische Analyse durch Histologie und Transmissionselektronenmikroskopie an denselben Versuchsobjekten durchgeführt werden. Das erfindungsgemässe Kontrastmittel verbleibt in den perfundierten Blutgefässen und ist autofluoreszent, was die "Lokalisierung" eines spezifischen histologischen Schnittes innerhalb des virtuellen Micro-CT-Schnitt-Stapels erleichtert.

Bezogen auf die Nierenmorphometrie, können weder bisherige Hochfeld-MRI-Techniken noch die vor Kurzem beschriebene Methode mit "Lightsheet Microscopy" mittels in vivo antiCD31-Markierung eine Darstellung des Gefässbaums der Niere in dem Ausmass bieten, wie das auf Micro-CT basierte Morphometrie-Verfahren mit dem erfindungsgemässen Kontrastmittel. Zudem ermöglicht dieses Verfahren eine 3D-Skeletonisierung und eine entsprechende Analyse des Organgefässsystems mittels bereits öffentlich verfügbarer Software. Neben der raschen 3D-Gefässdarstellung erlaubt das auf Micro-CT basierte Morphometrie-Verfahren eine grosse Zeiteinsparung (unter 24 Stunden gegenüber 1-2 Wochen bei dem klassischen, auf Histologie basierenden Verfahren oder der Ausgussmethode.

Überraschenderweise wurde festgestellt, dass das erfindungsgemässe Kontrastmittel auch zur Visualisierung von Knochengefässen nach der Entkalkung verwendet werden kann. Für die vorangehende Entkalkung, welche zum Stand der Technik gehört und nicht Gegenstand der Erfindung ist, können grundsätzlich drei Haupttypen von Entkalkungsmitteln verwendet werden: erstens solche, die auf starken Mineralsäuren basieren, wie beispielsweise Salzsäure oder Salpetersäure, zweitens solche, die auf schwächeren organischen Säuren basieren, wie beispielsweise Ameisensäure (z.B. in einer einfachen 10% wässrigen Lösung oder kombiniert mit Formalin oder mit einem Puffer) oder Trichloressigsäure, und drittens solche, welche aus sogenannten Chelatbildnern zusammengesetzt sind, z.B. eine 10% EDTA Lösung. Für den Zweck der Sichtbarmachung von Knochengefässen werden vorzugsweise Chelatbildner verwendet. EDTA (Ethylendiamintetraessigsäure) wirkt zwar langsam, aber bewirkt wenig Gewebezerstörung und herkömmliche Anfärbereagenzien werden kaum beeinflusst. Eine mögliche Zusammensetzung eines auf EDTA basierenden Entkalkungsmittels ist eine Mischung aus 250 g EDTA Dinatrium-Salz und 1750 ml destilliertem Wasser, wobei die Lösung auf pH 7 eingestellt wird, vorzugsweise durch Beigabe von ca. 25 g Natriumhydroxid.

Weitere Ausführungsbeispiele sind in den abhängigen Ansprüchen beschrieben.

### KURZE BESCHREIBUNG DER ZEICHNUNGEN

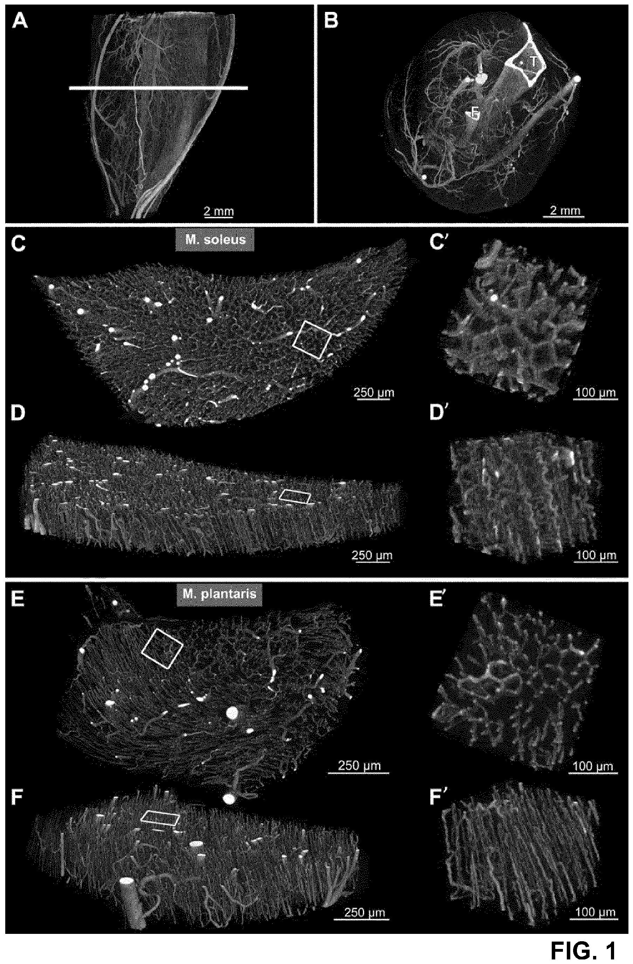

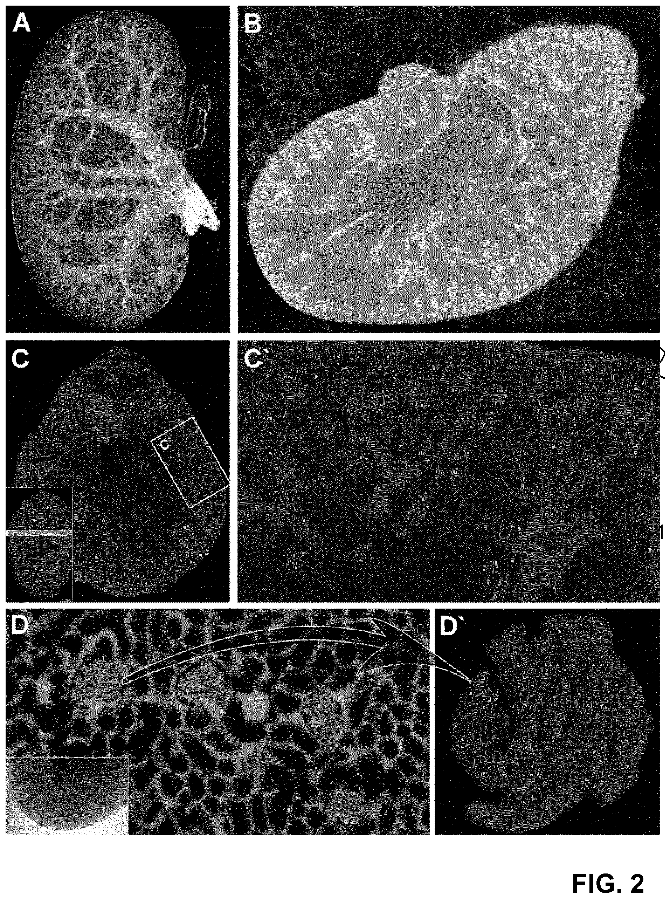

Das erfindungsgemässe Kontrastmittel fand bisher beispielsweise Einsatz bei der Darstellung der Vaskulatur der hinteren Extremität einer Maus (siehe Fig. 1), sowie der Nierenvaskulatur und Glomeruli (Fig. 2-5).

Bevorzugte Ausführungsformen, bzw. Beispiele für Anwendungen der Erfindung werden im Folgenden anhand der Zeichnungen beschrieben, die lediglich zur Erläuterung dienen und nicht einschränkend auszulegen sind. In den Zeichnungen zeigen:
- Fig. 1: Vaskulatur der unteren hinteren Extremität einer Maus, visualisiert durch MicroCT; wobei A) eine laterale 3D Ansicht zeigt, bei einer Voxel-Seitenlänge von 2.7µm; B) ein virtueller Transversalschnitt der Vaskulatur (auf dem in A angegebenen Niveau), bei einer Voxel-Seitenlänge von 0.8, bzw. 0.66µm: Tibia (T) und Fibula (F) scheinen leicht gefärbt aufgrund ihrer hohen Röntgenabsorption; in C-F sind virtuelle Transversalschnitte des isolierten Soleus Muskels (C, D) bzw. des Plantaris Muskels (E, F) dargestellt; in C')-F') sind spezifische Ausschnitte detaillierter dargestellt, markiert durch Rechtecke in C)-F); wobei die Mikrovaskulatur bei höherem Vergrösserungsgrad in Volumendarstellung abgebildet ist, mit unterschiedlicher Gefässdichte, Tortuosität, und 3D-Anordnung.
- Fig. 2: verschiedene Visualisierungsmodalitäten der Nierenvaskulatur und der Glomeruli; wobei in A) der rekonstruierte 3D-Stapel des Micro-CT-Datensatzes mit Fokus auf der Nierenvaskulatur dargestellt ist; In B ist ein virtueller Schnitt durch den Datensatz dargestellt, unter Verwendung einer anderen Transferfunktion: Die Visualisierung ist auf das Nierengewebe fokussiert; C-C' zeigen die Visualisierung mit Fokus auf die Glomeruli: Abbildung C zeigt ein Volumen-Rendering einer virtuellen 500 µm dicken Scheibe, wie durch die weisse Box unten links angezeigt; der weisse Rahmen in C zeigt die Stelle mit den Glomeruli in höherer Vergrösserung in Abbildung C' an; Abbildung D-D' stellt die fortgeschrittene Visualisierungsoption dar: Microangio-CT bei einer höheren Auflösung (Voxelseitenlänge =0.59µm). Der Einsatz in Abbildung D zeigt das in D gezeigte virtuelle Schnittniveau; Das 3D-Volumen-Rendering der Mikrovaskulatur eines in D markierten Glomerulus ist in D' dargestellt.

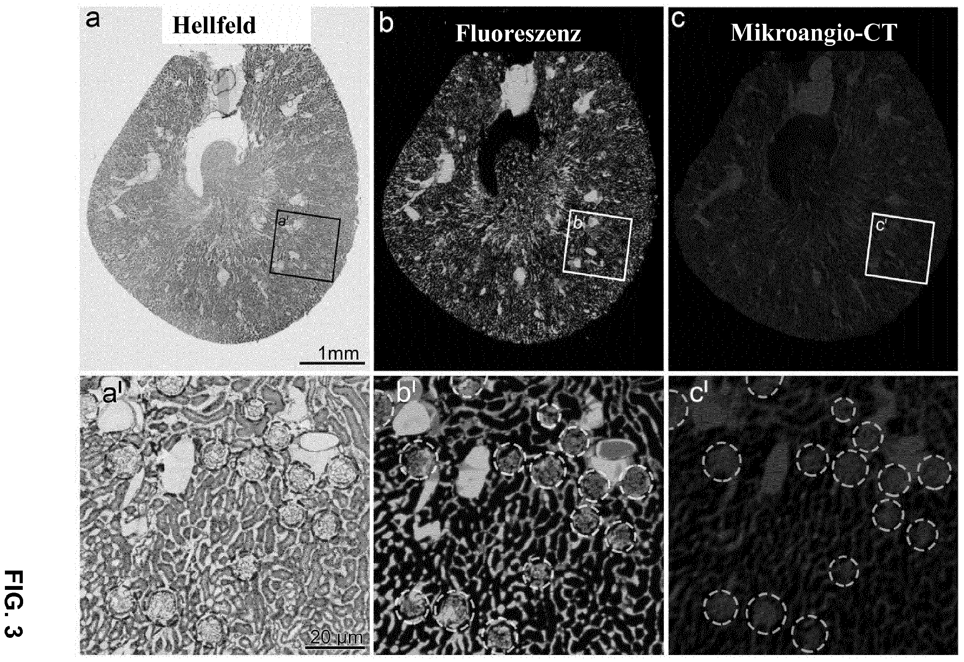

- Fig. 3: Korrelative Mikroskopie: Visualisierung von korrespondierenden Stellen unter Verwendung der Micro-CT-Daten und des histologischen Ansatzes. Nach der Bildgebung wurde die fixierte Niere zwecks histologischem Schnitt und Untersuchung weiter verarbeitet; Abbildungen a-c zeigen die Visualisierung desselben Niveaus (Schnittes) derselben Niere, unter Verwendung von Hellfeld- (a & a') und Fluoreszenz- (b & b') Mikroskopie, sowie Micro-CT (c & c'). Das grüne Signal in b und b' kommt vom autofluoreszierenden Kontrastmittel, welches in den Gefässen polymerisiert. Diese Eigenschaft erleichtert die Registrierung zwischen Histologie und Micro-CT aufgrund der Orientierung auf grössere Gefässe. In den Abbildungen a'-c' sind die in a-c markierten Regionen vergrössert dargestellt. Die Glomeruli sind mit Kreisen in a'-c' dargestellt.

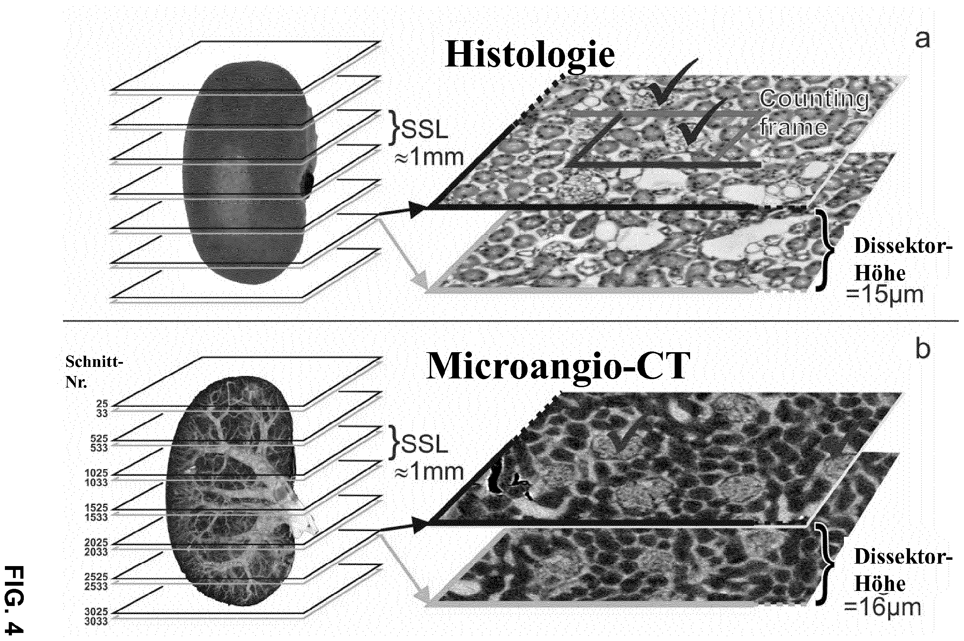

- Fig. 4.: Schema des angewandten kombinierten Fraktionator/Dissektor-Prinzips für stereologische Schätzung der Glomeruli-Zahl, basierend auf (a) histologischen und (b) Micro-CT Daten. a) Klassisches histologisches Prinzip: ausführliche Sektionierung der gesamten Niere, in Schnittpaaren bei 15µm Distanz (Dissektor Höhe (engl.: "disector height")) in ca. 10 equidistanten Niveaus (SSL: section sampling level, ca. 1mm). Schnittdicke 5µm. Für die Disektor-Höhe wird jeder dritte Schnitt verwendet, und für den SSL werden 197 Schnitte (200-3) weggeworfen, bis das nächste relevante Disektor-Paar verwendet wird. Auf den korrespondierenden Abbildungen, welche einen Disektor bilden, werden die Glomeruli gezählt, welche in einem Abschnitt und nicht im anderen auftauchen, und innerhalb des Zählrahmens liegen ohne die linke und untere Linie zu berühren (schwarze Häkchen). Die geschätzte Zahl wird durch den umgekehrten Schnittprobenfaktor (SSF) und Flächenprobenfaktoren (ASF) multipliziert, um die auf der Histologie basierende absolute Zahl von Glomeruli pro Niere zu erhalten (Nabs-histo(glom) (siehe Text).
b) Prinzip gemäss µaCT (Microangio-CT): Beinahe das gleiche Prinzip basierend auf virtuellen Bildstapeln, aus Micro-CT-Daten. Disektor Höhe (16µm oder 8 voxel Höhe) und Schnittmuster-Niveau (1mm oder 500 voxel Höhe) werden realisiert, indem die entsprechenden Schnittzahlen des Voxel-Stapels verwendet werden (linke Seite der Abbildung). Schnitt/Voxel-Höhe: 2µm. Alle Glomeruli auf einem gesamten Schnittniveau wurden nach dem Disektor-Prinzip gezählt (Häkchen), sodass kein Zählrahmen nötig ist. Die geschätzte Glomeruli-Zahl wird nur mit dem umgekehrten Schnittmuster-Faktor multipliziert, wodurch kein Bedarf für den Flächenmuster-Bruchteil besteht, um die gesamte Zahl der Glomeruli pro Niere zu erhalten (Nabs-µaCT (glom)).

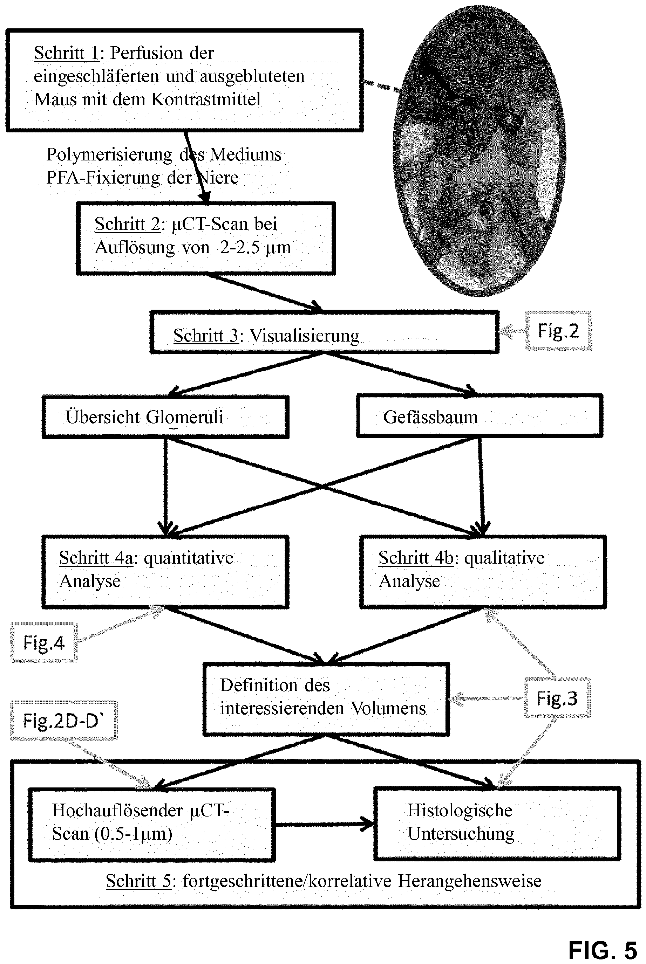

- Fig. 5.: Schema des vorgeschlagenen Mikroangio-CT-Verfahrens der Niere (nach Fig. 2-4) unter der Verwendung des erfindungsgemässen Kontrastmittels.

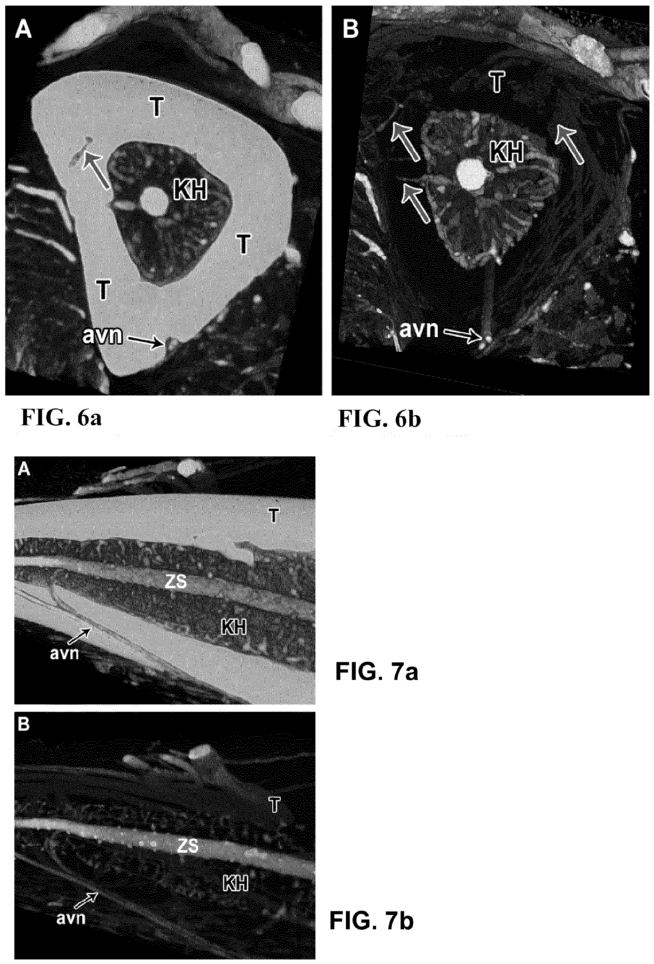

- Fig. 6.: Vaskulatur des Tibiaknochens einer Maus, dargestellt mittels microCT unter Anwendung des erfindungsgemässen Kontrastmittels; (6a) und (6b) zeigen beide einen virtuellen Querschnitt durch denselben Tibiaknochen, wobei in (6a) der Knochen vor und in (6b) nach der Entkalkung mit EDTA 10% abgebildet ist. Aufgrund der höheren Röntgenabsorption scheint Tibiaknochen (T) in (6a) heller, und in (6b) infolge der niedrigen Röntgenabsorption nach der Entkalkung transparent. Demzufolge sind die kleinen, quer durch Knochengewebe durchlaufende Verbindungsgefässe zwischen Periostalgefässen und Gefässen der Knochenmarkhöhle (KH) in (6b) besser sichtbar und auffindbar (aufwärts gerichtete Pfeile). Die Visualisierung der Gefässe innerhalb der Knochenmarkhöhle ist infolge der geringeren Röntgenabsorption des entkalkten Knochengewebes der Tibia in (6b) sichtbar besser. Am äusseren Rande der Tibia sind die Versorgungsarterien (avn=arteria und vena nutricia) gut erkennbar.
- Fig. 7.: Vaskulatur des Tibiaknochens einer Maus, dargestellt mittels microCT unter Anwendung des erfindungsgemässen Kontrastmittels; (7a) und (7b) zeigen beide den virtuellen Längsschnitt durch denselben Tibiaknochen, wobei in (7a) der Knochen vor und in (7b) nach der Entkalkung mit EDTA 10% abgebildet ist. Aufgrund der höheren Röntgenabsorption scheint der Tibiaknochen (T) in (7a) heller, in (7b) infolge der niedrigen Röntgenabsorption nach der Entkalkung transparent. Demzufolge sind die durch Knochengewebe hindurch laufenden Knochengefässe (avn=arteria und vena nutricia) in (7b) einfacher zu verfolgen, obwohl sie auch in (7a) sichtbar sind. In der Mitte der Knochenmarkhöhle (KH) ist der Zentrale Sinus (ZS) mit seinen Verbindungen gut sichtbar.

### BESCHREIBUNG BEVORZUGTER AUSFÜHRUNGSFORMEN

### 1. Vorbereitung der einzelnen Komponenten:

Es werden 3 Behälter bereitgestellt. Behälter 1 enthält eine erste Mischung aus iodiertem, verestertem Öl, vorzugsweise iodiertes, verestertes Leinsamenöl (Leinöl) und 2-Butanon (C₄H₈O), und einen Farbstoff (BlueDye von VasQtec). Diese erste Mischung, ob mit oder ohne Farbstoff, wird im Rahmen dieser Anmeldung "Kontrastlösung" genannt. Behälter 2 enthält das Polyurethan (PU). Behälter 3 enthält den Härter.

### 2. Entfernung der Kontrastlösung aus dem Behälter 1:

Aufschrauben einer 12ml-Einwegspritze (z.B. Monoject syringe with luer lock c1086, Qosina) auf Luer Connector (Needlefree Swabable Valve Female Luer to 20mm Vial Cap Polycarbonate, Value Plastic) des Behälters 1; Einspritzen von 5 ml Luft in den Behälter 1 (aufrechter Druck), Umdrehen des Behälters 1, Aspiration der kompletten Kontrastlösung in die Spritze.

### 3. Herstellung einer zweiten Mischung aus Kontrastlösung und Polyurethan:

Einspritzen der Kontrastlösung in den Behälter 2 (welcher das PU enthält); Entfernung der Spritze und Verschliessen des Luer Connectors mit Luer Kappe.

### 4. Mischung der zweiten Mischung:

Mischen der zweiten Mischung in Behälter 2 auf einem Vortex-Gerät. Die Viskosität der für die bisherigen Versuche verwendeten zweiten Mischung aus Kontrastlösung und PU (ohne Härter) beträgt ca. 100 mPas.s bei 20°C.

### 5. Aspiration der zweiten Mischung in einer Spritze:

Entfernung der Luer Kappe; Einspritzen von 10 ml Luft in den Behälter 2 Umdrehen des Behälters 2 und Aspiration der zweiten Mischung in eine 12ml-Einwegspritze.

### 6. Aspiration des Härters:

Aufschrauben einer lml-Einwegspritze (1 ml syringe with luer slip c3302, Qosina) auf den Behälter 3 (bzw. auf einen auf Behälter 3 befestigten Luer Connector); Einspritzen von 0.5 ml Luft in den Behälter 3; Aspiration des gesamten Härters (1 ml).

### 7. Lagerung/Entfernung von Mikrobläschen:

Zur Entlüftung der Spritzeninhalte, d.h. zur Entfernung von Mikrobläschen, werden die Spritzen für mindestens 15 min in eine aufrechte Position gebracht.

### 8. Vorbereitung der Maus/Ratte:

Zunächst wird das Tier tief betäubt oder euthanasiert und dann kanüliert. Das Ausspülen des Tierkörpers erfolgt mit 2 x 5-50 ml Klarlösung, vorzugsweise mit isotonischer Lösung, wie beispielsweise PBS (phosphate buffered saline) Lösung. Vorzugsweise wird das Tier in zwei Hälften ausgespült (und dann auch jede Hälfte einzeln mit Kontrastmittel gefüllt).
8a.) Zwecks Kanülierung und Ausspülung der *unteren* Körperhälfte wird eine Kanüle (z.B. BD Neoflon 0.6x19 mm, 26G, von Aichele Medica AG) in die Aorta eingeführt, mit anschliessender antegrader Perfusion der Klarlösung, d.h. vom Herz weg. Der Einstichort in die Aorta befindet sich vorzugsweise im Thorakalbereich der Aorta. Ein Indiz für die Füllung der unteren Körperhälfte ist das Ballonieren des Herzens: Wenn das Herz beginnt, zu ballonieren, wird eine Inzision im rechten Vorhof vorgenommen. Dort läuft die Klarlösung dann aus, zunächst vermischt mit dem ausgespülten Blut des Tieres. Sobald die aus der Vorhof-Inzision austretende Klarlösung klar heraustritt, kann davon ausgegangen werden, dass die untere Körperhälfte vollständig gespült ist.
8b.) Zwecks Kanülierung der *oberen* Körperhälfte wird eine Kanüle, am selben Einstichort eingeführt, allenfalls die gleiche Kanüle wie in Schritt 8a in umgekehrter Ausrichtung, wobei im gleichen Gefäss, d.h. in der Aorta, mit der Klarlösung retrograd, d.h. gegen das Herz, perfundiert wird.

### 9. Befestigung der Spritze:

9a.) am (manuellen) Dispenser: Einsetzen der 12ml-Spritze und der lml-Spritze mit ihrem jeweiligen ersten Ende in einen 2-Spritzen-Dispenser (zwei zusammengefügte Einwegdispenser 11:1, M-System, Medmix Systems AG); Befestigen eines Adapters (Adapter L-System, Medmix Systems AG) auf das jeweilige zweite Ende der beiden Spritzen; Befestigung des Adapters an einem Mischbehälter (Mischer, ML 3.2-16-LLM, DN3.2x16, Med.LuerLock, Medmix Systems AG); oder
9b.) an der Injektionspumpe: Befestigung beider Spritzen auf der Injektionspumpe; manuelles Einstellen der richtigen Position der Pumpe; Aufschrauben des Adapters auf das jeweilige zweite Ende der beiden Spritzen; Befestigung des Adapters am Mischbehälter; Einrichtung der Pumpe (Syringe Pump LEGATO 100, 220V/50 Hz, CE, kdScientific), Wahl der Parameter (Modus: Infuse only; Syringe: Sherwood 12ml; Flussraten: max. 1.5 ml/min; Maximalvolumen: ca. 3 ml/Maus, ca. 9-10 ml/ Ratte)

### 10. Injektion/Perfusion:

10a.) sorgfältig und gleichmässig, mit möglichst gleichmässigem Fluss, oder
10b.) Pumpe in Betrieb nehmen, Flussrate max. 1.5 ml/min

### Füllung der gesamten unteren und/oder oberen Körperhälfte des Tierkörpers:

Nun wird eine Kanüle (z.B: BD Neoflon 0.6x19 mm, 26G, von Aichele Medica AG, ggf. die für die Ausspülung verwendete Kanüle) am manuellen Dispenser (Variante a) oder an der Pumpe (Variante b) befestigt.

Zur Perfusion der *unteren* Körperhälfte wird, wie bei der Auswaschung, das Kontrastmittel durch eine antegrade Perfusion vom gleichen Einspritzort aus, der für die Ausspülung bzw. Desanguisierung des Tieres gedient hat, in den Tierkörper in Richtung vom Herzen weg eingespritzt. Als Indiz für die Füllung der *unteren* Körperhälfte dient die Verfärbung der unteren Extremitäten in der Farbe des Kontrastmittels (vorzugsweise blauer Farbstoff).

Zur Füllung der gesamten *oberen* Körperhälfte mit dem Kontrastmittel wird nun, wie bei der oben beschriebenen Kanülierung bzw. Auswaschung, am gleichen Einspritzort, das Kontrastmittel in die obere Körperhälfte des Tierkörpers eingespritzt, d.h. durch eine retrograde Perfusion des Kontrastmittels. Zur Absicherung, dass kein Kontrastmittel ins Herz läuft, d.h. um eine Dilatation des linken Ventrikels zu verhindern, wird die Aorta ascendens mit einem Faden abgebunden. Wenn die oberen Extremitäten, d.h. die Pfoten, sowie die Nase des Tieres die Farbe des Kontrastmittels annehmen, kann davon ausgegangen werden, dass die obere Körperhälfte des Tieres komplett gefüllt ist.

Zur Füllung eines gesamten Körpers einer Maus bzw. einer Ratte sind bis zu ca.10 ml bzw. bis zu ca. 35 ml Kontrastmittel nötig.

### Selektive Füllung eines oder mehrerer Organe des Tierkörpers:

Alle Organe, welche der unteren Körperhälfte angehören, können wie oben beschrieben durch die gesamte Füllung der unteren Körperhälfte mit Kontrastmittel gefüllt werden. Das Gehirn kann durch die gesamte Füllung der oberen Körperhälfte mit Kontrastmittel gefüllt werden.

Das Füllen des Herzens und/oder der Lunge muss aber selektiv durchgeführt werden: dazu wird eine Kanüle in die Aorta descendens eingeführt, wobei distal von der Aorta descendens abgeklemmt wird, damit nur die Aorta ascendens und die Herzkranzgefässe gefüllt werden. Dann wird Klarlösung (z.B. PBS) nur in diesen abgeklemmten Teil eingespritzt.

Das Lungengefässsystem wird über die Lungenvenen, welche in den linken Vorhof des Herzens münden, retrograd gefüllt.

Zum Füllen selektiver Organe, z.B. des Herzens und/oder der Lunge sind, im Vergleich zur vollständigen Füllung des Tierkörpers, nur ca. 0.5-1.5 ml Kontrastmittel nötig.

### 11. Aushärtenlassen:

Nach dem Einspritzen wandert das Kontrastmittel von der Aorta über die Arterien in das Kapillarnetz und venöses System des Tierkörpers hinein und härtet dort infolge Polymerisation aus. Das Kontrastmittel sollte mindestens 20-30 min aushärten. Anschliessend wird der Tierkörperteil mit ausgehärtetem Kontrastmittel chemisch fixiert, vorzugsweise mit 2% Paraformaldehyd-Lösung, und ist dann bei 0-8°C lagerbar bis zu mehreren Monaten.

### 12. Bildanalyse mittels Micro-CT-Scan:

Das Bildgebungsverfahren des Tierkörpers, bzw. das Scannen mittels Micro-CT-Gerät, erfolgt im ausgehärteten Zustand. Während des Scannens darf sich der Körper nicht bewegen/bewegt werden, da dies zu Störungen der Aufnahmen führt. Zur Verhinderung von kleinsten Bewegungen sollte der Tierkörper während des Scans mechanisch fixiert werden.

Die Versuchsobjekte wurden für die in den Figuren dargestellten Bilder unter Verwendung eines "desktop microCT" Gerätes (SkyScan 1172 oder 1272, Bruker, MicroCT, Kontich, Belgien) gescannt.

### 13. Lagerung:

Nach Ausführung des CT-Scans kann der Tierkörper wieder in einer 2%-Paraformaldehyd-Lösung bei 0-8°C gelagert werden.

### 14. Histologie:

Anschliessend können histologische Untersuchungen von Tierkörperteilen oder Organen vorgenommen werden. Dazu sind die klassische Paraffin-Einbettung, die klassische histologische Schnitttechnik, Färbungen sowie Immunohistochemie anwendbar.

Dabei verbleibt das ausgehärtete Kontrastmittel intravaskulär und ist gut sichtbar, auch nach der histologischen Sektion. Die Autofluoreszenz des Kontrastmittels erlaubt einen direkten Vergleich der histologischen Schnitte mit den korrespondierenden virtuellen Schnitten des Micro-CT-Datensatzes.

### 15. Auswertung (Quantitative und qualitative Analyse des Micro-CT-Datensatzes):

MicroCT Projektionen können nach dem Scannen rückwärts projektionsrekonstruiert werden, z.B. unter Verwendung der NRecon Software (NReconServer64bit, Bruker, MicroCT, Kontich, Belgien), "volume-gerendert" und mittels der CTVox Software (Bruker, MicroCT, Kontich, Belgium) dreidimensional visualisiert werden. Gewebe und Blutgefässe können segmentiert und analysiert werden unter Verwendung der CTAn Software (Bruker, MicroCT, Kontich, Belgium) oder auch anderer öffentlich verfügbarer Software, wie z.B. Matlab (The MathWorks, Inc., Natick, MA, USA) oder ImageJ (Rasband, W.S., ImageJ, U. S. National Institutes of Health, Bethesda, Maryland, USA, http://imagej.nih.gov/ij/, 1997-2016.) bestimmt.

### Mischtests:

Bei der Suche nach der optimalen Zusammensetzung des Kontrastmittels wurden verschiedene Verhältnisse des iodierten, veresterten Leinöls zum 2-Butanon und zum PU getestet. Dabei wurde stets das bevorzugte konstante Verhältnis von PU zu Härter von 100:16 Gewichtsprozent verwendet. Zudem wurden jeweils 0.2 g (bzw. eine Messerspitze) eines blauen Farbstoffs in Pulverform verwendet. Die Mischtests wurden wie folgt durchgeführt:
Jede Komponente wurde einzeln bereitgestellt. Das Polyurethan (PU) wurde mit dem Butanon, dem iodierten, veresterten Leinöl, und dem Farbstoff unter Verwendung eines Ultraschall-Bads in einem Glasbehälter gemischt. Anschliessend wurde der Härter beigefügt, und während ca. 30 sec. ebenfalls im Ultraschallbad gemischt. Der Glasbehälter mit der Mischung wurde dann in eine Vakuumkammer gestellt und gewartet, bis kleine Bläschen sich an der Oberfläche bildeten (ca. 40 sec.). Anschliessend wurde eine Spritze mit der Mischung gefüllt und die Mischung in das zu untersuchende Objekt injiziert (Perfusion).

Zur Bestimmung der Viskosität wurde die Mischung einem "Drop Fall Test" unterzogen. Jede Minute wurden 0.1ml der Mischung auf ein Blatt Papier, welches in vertikaler Position gehalten wurde, aufgetragen. Die Mischung durchlief das Papier. Um die Viskosität zu messen, wurde die Distanz, welche von der Mischung zu bestimmten Zeitpunkten passiert wurde, beobachtet. Ein Venflon-Venenkatheter wurde auf der Spritze montiert, um die Perfusion am Körper zu imitieren. Nach dem Viskositätstest wurde der Venenkathether mit der polymerisierten Mischung von der Spritze entfernt. Alle Venenkatheter wurden in einem micro-CT-Gerät auf die Absorption der verschiedenen Mischungen untersucht.

| **Probe Nr.** | **PU** | **Härter** | **2-Butanon** | **iod.,verestertes Leinöl** | **Farbstoff** | **Bemerkung** |
|---|---|---|---|---|---|---|
| 1a | 5g | 0.8ml | 2ml | 2.2ml | 1 Messerspitze | Minimum |
| 1b | 5g | 0.8ml | 2.2ml | 2.5ml | 1 Messerspitze | |
| 1c | 5g | 0.8ml | 2.2ml | 3.0ml | 1 Messerspitze | |
| 2 | 5g | 0.8ml | 2ml | 3.0ml | 1 Messerspitze | |
| 3 | 5g | 0.8ml | 1ml | 3.0ml | 1 Messerspitze | keine Perfusion |
| 4 | 5g | 0.8ml | 1.5ml | 3ml | 1 Messerspitze | keine Perfusion |
| 5 | 5g | 0.8ml | 1.5ml | 3.5ml | 1 Messerspitze | Keine Perfusion |
| 6 | 5g | 0.8ml | 2ml | 3.5ml | 1 Messerspitze | Abscheidung |
| 7 | 5g | 0.8ml | 2ml | 5ml | 1 Messerspitze | Abscheidung |
| 8 | 5g | 0.8ml | 2ml | 7ml | 1 Messerspitze | Abscheidung |
| 9 | 5g | 0.8ml | 2ml | 8ml | 1 Messerspitze | Abscheidung; max. Öl |

In Bezug auf die Polymerisation eigneten sich alle bis auf drei der getesteten Zusammensetzungen für die Perfusion. Es wurden 10 Minuten als Minimalzeit für die Perfusion festgelegt. Die Proben la-c, 2, 6-9 erfüllten diese Voraussetzung.

Die Tests zeigten, dass mindestens 2 ml 2-Butanon (für 5g PU und 0.8ml Härter) verwendet werden sollten. Unter diesem Wert erfolgt die Polymerisation zu schnell und erlaubt daher keine vollständige Perfusion.

Die Menge des iodierten, veresterten Öls beeinflusst ebenfalls die Polymerisationszeit. Die Proben 8 und 9 zeigten eine schnellere Polymerisation als die anderen Proben. Daher scheint es, als ob bei unverändertem Volumen des PU, des Härters und des 2-Butanons, 8ml des Öls der maximalen geeigneten Konzentration entsprechen.

Proben 6-9 zeigten eine Abscheidung von Öl und Farbstoff nach Beendung der Polymerisation, was zur Diffusion des Kontrastmittels in das umliegende Gewebe, und möglicherweise zur Verminderung der Bildqualität führen könnte.

Proben 8 und 9 wiesen eine hohe Konzentration des iodierten Öls auf und somit auch eine hohe Absorption (ev. ähnlich wie Knochengewebe). Dies erfordert zur Reduktion der Artifakte die Verwendung eines Aluminium-Filters für das Scannen, was zu einer längeren Scan-Dauer führt. Eine hohe Absorption könnte allerdings auch die Kapillar-Erkennung positiv beeinflussen, könnte aber auch zu einer Übersättigung der Kapillar-Pixel führen, was den Partialvolumeneffekt vermindern würde und möglicherweise eine grössere PixelGrösse erlauben würde (in den Versuchen wurde jeweils eine isotrope Pixelgrösse von 0.8 µm verwendet).

Die Verwendung von iodiertem, verestertem Mohnöl anstatt von iodiertem, verestertem Leinöl zeigte eine gute Perfusion, aber einen schlechteren Kontrast bei der Angiographie, was wahrscheinlich auf den geringeren Iodanteil zurück zu führen ist. Die Verwendung von Aceton statt Butanon als Lösungsmittel zeigte ähnliche Effekte wie Butanon, was auch von anderen Ketonen, wie beispielsweise Diethylketon zu erwarten ist. Auch eine Verwendung von Methylenchlorid als Lösungsmittel-Alternative ist denkbar.

Folgende Messungen dienten dem Ausdruck der Konzentrationen als Gewichtsprozente in der untenstehenden Tabelle: 3.5 ml iodiertes, verestertes Leinöl wogen 4.8g. 0.8 ml Härter wogen 0.8g. 2 ml Butanon wogen 1.5g. Das verwendete PU hatte eine Dichte von ca. 1.05 g/cm³:

| Probe | PU | Härter | Butanon | iod.,verestertes Leinöl |
|---|---|---|---|---|
| 1a | 48.48% | 7.76% | 14.54% | 29.22% |
| 1b | 45.96% | 7.35% | 15.17% | 31.52% |
| 1c | 43.24% | 6.92% | 14.27% | 35.56% |
| 2 | 43.82% | 7.01% | 13.15% | 36.02% |
| 3 | 46.90% | 7.50% | 7.04% | 38.56% |
| 4 | 45.31% | 7.25% | 10.19% | 37.25% |
| 5 | 42.66% | 6.83% | 9.60% | 40.91% |
| 6 | 41.34% | 6.61% | 12.40% | 39.64% |
| 7 | 35.34% | 5.65% | 10.60% | 48.41% |
| 8 | 29.60% | 4.74% | 8.88% | 56.78% |
| 9 | 27.38% | 4.38% | 8.21% | 60.02% |

### Optimierungstests :

Nach den Mischtests wurde die Kontrastmittel-Mischung weiterhin optimiert. Beim aktuellen, oben beschriebenen Verfahren zum Einspritzen des Kontrastmittels in den zu untersuchenden Körper bzw. das zu untersuchende Organ oder Gewebe wird ja der Härter, bzw. der Inhalt des 3. Behälters erst beim bzw. unmittelbar vor dem Einspritzen den restlichen Kontrastmittelkomponenten beigemischt. Dazu wird eine Doppelspritze verwendet. Diese gibt ein gewisses Volumenverhältnis von 1:11 zwischen dem Härter und der restlichen (zweiten) Mischung vor. Daher ergibt sich stets ein Härterüberschuss, weshalb die Menge des Härters an der Gesamtmenge nicht definierend sein sollte. Bei den Mischtests wurde noch keine Doppelspritze verwendet, und daher wurde eine definierte Menge Härter von 0.8 ml verwendet.

Der bevorzugte Bereich des Volumenverhältnisses vom PU zum Härter beträgt 100:16-100:19. Dies ist aber auch variabel und beeinflusst die Qualität des Kontrastmittels nicht substantiell.

Bei den Optimierungstests wurde in der Kontrastlösung (bei Vernachlässigung des optionalen Farbstoffs aufgrund dessen kleiner Menge) ein Volumenverhältnis vom iodierten, veresterten Leinöl zum 2-Butanon von 54%/46% als optimal befunden (Varianten 2, 5, 6). Aber auch Volumenverhältnisse vom iodierten, veresterten Leinöl zum 2-Butanon von 53%/47% (Variante 1) oder von 56%/44% (Variante 3) oder gar 58%/42% (Variante 4) zeigen gute Resultate bei der Perfusion und anschliessend einen guten Kontrast bei der Bildgebung. Somit lassen sich bevorzugte Bereiche der Verhältnisse vom Volumen des iodierten, veresterten Öls zum Volumen des Ketons definieren, nämlich 0.75-4, vorzugsweise 1-1.5, insbesondere 1.1-1.3.

Das iodierte, veresterte Öl beeinflusst den Kontrast. Das Butanon dient als Verflüssigungsmittel. Für einen gewünschten höheren Kontrast wird der Mischung verhältnismässig mehr iodiertes, verestertes Öl beigemischt, für weniger Kontrast entsprechend weniger iodiertes, verestertes Öl. Bei einer verhältnismässig zu grossen Ölmenge kommt es zum Austreten von Öl aus der Lösung aufgrund von Übersättigung und zu einer zu hohen Viskosität, was die Perfusion erschwert bzw. verhindert.

Da beim Entleeren der einzelnen Behälter und beim Mischen der Komponenten sowie beim Einspritzen des Kontrastmittels sowohl in den Behältern, als auch in den Mischröhrchen und den Spritzen jeweils ein Restvolumen an den Wänden bzw. am Behälterboden verbleibt, wurden die Volumina der Komponenten in den Optimierungstests bei gleichbleibendem optimalen Verhältnis von iodiertem, veresterten Leinöl zu 2-Butanon (54%/46% von Variante 2) auf das Füllmaximum der Behälter optimiert (Varianten 5, 6). Die effektiven Füllmengen der Behälter sind somit natürlich vom jeweiligen Behältervolumen bzw. an die vom zu untersuchenden Objekt abhängige Füllmenge anzupassen.

**Varianten der Zusammensetzung des Kontrastmittels:**

| **Kontrastmittel-Kit (Variante 1: Minimum)** | | | | |
|---|---|---|---|---|
| **Behälter Nr.** | | **Menge [ml]** | **Mengen der Kontrastlösungs-Komponenten [ml]** | **Volumenprozent** [%] (vom Total) |
| 1 | **Kontrastlösung:** (iod. Leinöl, 2-Butanon, Farbstoff) | 4.70 | | |
| | | | 2.50 | 24.39 |
| | | | 2.20 | 21.46 |
| | | | 1 Messerspitze | vernachlässigbar |
| 2 | **PU** | 4.75 | | 46.34 |
| 3 | **Härter** | 0.80 | | 7.80 |
| Total | | **10.25** | | **100** |

| **Kontrastmittel-Kit (Variante 2: Optimum)** | | | | |
|---|---|---|---|---|
| **Behälter Nr.** | | **Menge [ml]** | **Mengen der Kontrastlösungs-Komponenten [ml]** | **Volumenprozent** [%] (vom Total) |
| 1 | **Kontrastlösung:** (iod. Leinöl, 2-Butanon, Farbstoff) | 4.80 | | |
| | | | 2.60 | 25.12 |
| | | | 2.20 | 21.26 |
| | | | 1 Messerspitze | vernachlässigbar |
| 2 | **PU** | 4.75 | | 45.89 |
| 3 | **Härter** | 0.80 | | 7.73 |
| Total | | **10.35** | | **100** |

| **Kontrastmittel-Kit (Variante 3:Maximum)** | | | | |
|---|---|---|---|---|
| **Behälter Nr.** | | **Menge [ml]** | **Mengen der Kontrastlösungs-Komponenten [ml]** | **Volumenprozent** [%] (vom Total) |
| 1 | **Kontrastlösung:** (iod. Leinöl, 2-Butanon, Farbstoff) | 5.00 | | |
| | | | 2.80 | 26.54 |
| | | | 2.20 | 20.85 |
| | | | 1 Messerspitze | vernachlässigbar |
| 2 | **PU** | 4.75 | | 45.02 |
| 3 | **Härter** | 0.80 | | 7.58 |
| Total | | **10.55** | | **100** |

| **Kontrastmittel-Kit (Variante 4, mit angepasster Füllmenge, inkl. Verlustvolumen)** | | | | |
|---|---|---|---|---|
| **Behälter Nr.** | | **Menge [ml]** | **Mengen der Kontrastlösungs-Komponenten [ml]** | **Volumenprozent** [%](vom Total) |
| 1 | **Kontrastlösung:** (iod. Leinöl, 2-Butanon, Farbstoff) | 6.3 | 3.65 | 27.65 |
| | | | 2.65 | 20.08 |
| | | | 1 Messerspitze | vernachlässigbar |
| 2 | **PU** | 5.80 | | 43.94 |
| 3 | **Härter** | 1.10 | | 8.33 |
| Total | | **13.20** | | **100** |

| **Kontrastmittel-Kit (Variante 5: Optimum mit angepasster Füllmenge)** | | | | |
|---|---|---|---|---|
| **Behälter Nr.** | | **Menge [ml]** | **Mengen der Kontrastlösungs-Komponenten [ml]** | **Volumenprozent** [%](vom Total) |
| 1 | **Kontrastlösung:** (iod. Leinöl, 2-Butanon, Farbstoff) | 5.20 | 2.80 | 26.05 |
| | | | 2.40 | 22.33 |
| | | | 1 Messerspitze | vernachlässigbar |
| 2 | **PU** | 4.75 | | 44.19 |
| 3 | **Härter** | 0.80 | | 7.44 |
| Total | | **10.75** | | **100** |

| **Kontrastmittel-Kit (Variante 6: Optimum mit angepasster Füllmenge, inkl. Verlustvolumen)** | | | | |
|---|---|---|---|---|
| **Behälter Nr.** | | **Menge [ml]** | **Mengen der Kontrastlösungs-Komponenten [ml]** | **Volumenprozent** [%] (vom Total) |
| 1 | **Kontrastlösung:** (iod. Leinöl, 2-Butanon, Farbstoff) | 6.30 | 3.40 | 25.76 |
| | | | 2.90 | 21.97 |
| | | | 1 Messerspitze | vernachlässigbar |
| 2 | **PU** | 5.80 | | 43.94 |
| 3 | **Härter** | 1.10 | | 8.33 |
| Total | | **13.20** | | **100** |

**Viskositäten:**

| **Komponente** | **Viskosität** ([mPa s/ 20°C] |
|---|---|
| Iodiertes, verestertes Leinöl | 85 |
| 2-Butanon | 0.4 |
| PU | 6500 |
| Härter | 290 |

Die Viskosität der zweiten Mischung, d.h. der Kombination aus Kontrastlösung und PU (bzw. des Kontrastmittels noch ohne Härter) beträgt ca. 100 mPas.s bei 20°C.

### REFERENZEN

- Zagorchev, L. et al., Micro computed tomography for vascular exploration, Journal of Angiogenesis Research 2010, 2:7
- Meyer, E. et al., Polyurethane Elastomer: A New Material for the Visualization of Cadaveric Blood Vessels, Clinical Anatomy 20:000-000 (2007) (Wiley Interscience)
- Krucker et al., New Polyurethane-Based Material for Vascular Corrosion Casting with Improved Physical and Imaging Characteristics. Res. Tech. 69: 138-147 (2006)
- Meyer, E. et al., Altered morphology and 3D architecture of brain vasculature in a mouse model for Alzheimer's disease, PNAS, 105; 9; 3587-3592 (2008)
- Grabherr et al., Angiofil®-mediated Visualization of the Vascular System by Microcomputed Tomography: A Feasibility Study, Microscopy Research and Technique 71(7): 551-556, 2008.
- Shokiche, C.C., Baumann P., Hlushchuk R., Djonov V., Reyes M. (2016) High-Throughput Glomeruli Analysis of µ-CT Kidney Images Using Tree Priors and Scalable Sparse Computation. In: Ourselin S., Joskowicz L., Sabuncu M., Unal G., Wells W. (eds) Medical Image Computing and Computer-Assisted Intervention - MICCAI 2016.
- Perrien, D.S. et al., Novel methods for microCT-based analyses of vasculature in the renal cortex reveal a loss of perfusable arterioles and glomeruli in eNOS-/- mice, BMC. Nephrol. 17, 24 (2016).
- Schaad, L., et al., Correlative Imaging of the Murine Hind Limb Vasculature and Muscle Tissue by MicroCT and Light Microscopy, Scientific Reports, 7:41842 (2017), doi: 10.1038/srep4184
- WO 2008/034270 A2 (FORIM X AG [CH]; GRABHERR SILKE [CH]) 27. März 2008
- WO 2009/081169 A2 (IOPHARMA TECHNOLOGIES AB [SE]; WANG JIAN SHENG [SE]; KIDD SARA [GB]; A) 2. Juli 2009

## Patentansprüche

1. Kontrastmittel für *ex vivo* Mikroangiografie, vorzugsweise zur digitalen Bildgebung eines Gefässsystems einer Maus oder einer Ratte oder anderer Labortiere, oder einzelner tierischer und menschlicher Organe, mittels eines Mikro-CT-Geräts, aufweisend
- ein Polyurethan, und
- einen Härter,
**dadurch gekennzeichnet, dass** das Kontrastmittel zudem
- iodiertes, verestertes Öl, und
- ein Keton
enthält.

2. Kontrastmittel nach Anspruch 1, **dadurch gekennzeichnet, dass** das iodierte, veresterte Öl ein iodiertes, verestertes Mohnsamenöl, oder ein iodiertes, verestertes Leinsamenöl ist, wobei das iodierte, veresterte Leinsamenöl vorzugsweise Ethyl-9, 12, 15-triiodo-octadecatrienoat ist.

3. Kontrastmittel nach einem der vorhergehenden Ansprüche, **dadurch gekennzeichnet, dass** das Keton ausgewählt ist aus 2-Butanon, Aceton oder 3-Pentanon, wobei das Keton vorzugsweise 2-Butanon ist.

4. Kontrastmittel nach einem der vorhergehenden Ansprüche, **dadurch gekennzeichnet, dass** das Kontrastmittel einen Farbstoff enthält, wobei der Farbstoff vorzugsweise ein blauer Farbstoff ist.

5. Kontrastmittel nach einem der vorhergehenden Ansprüche, **dadurch gekennzeichnet, dass** das Polyurethan ein Polyisocyanat-Präpolymer ist, vorzugsweise ein aliphatisches Isocyanat, insbesondere bevorzugt 4,4'-Methylendi(cyclohexyl-isocyanat).

6. Kontrastmittel nach einem der vorhergehenden Ansprüche, **dadurch gekennzeichnet, dass** der Härter ein modifiziertes aromatisches Diamin ist, wobei der Härter vorzugsweise ein Diethylmethylbenzoldiamin ist, insbesondere bevorzugt eine Mischung von zwei Isomeren von Diethylmethylbenzoldiamin ist, am meisten bevorzugt ein Isomerengemisch von 2,6-Diamino-3,5-diethyltoluol und 2,4-Diamino-3,6-diethyltoluol im Verhältnis 7:3.

7. Kontrastmittel nach einem der vorhergehenden Ansprüche, **dadurch gekennzeichnet, dass**
- das iodierte, veresterte Öl zu 20-60%, vorzugsweise zu 22-45%, insbesondere bevorzugt zu 24-30% im Kontrastmittel enthalten ist; und
- das Keton zu 7-30%, vorzugsweise zu 10-25%, insbesondere bevorzugt zu 14-22% im Kontrastmittel enthalten ist.

8. Kontrastmittel nach einem der vorhergehenden Ansprüche, **dadurch gekennzeichnet, dass** in einer Kontrastlösung, welche das iodierte, veresterte Öl und das Keton aufweist, das Verhältnis vom Volumen des iodierten, veresterten Öls zum Volumen des Ketons 0.75-4 beträgt, vorzugsweise 1-1.5, insbesondere 1.1-1.3 beträgt.

9. Kontrastmittel nach einem der vorhergehenden Ansprüche, **dadurch gekennzeichnet, dass** das Polyurethan zu 25-60%, vorzugsweise zu 35-55%, insbesondere bevorzugt zu 38-50%, und am meisten bevorzugt zu 43-47% im Kontrastmittel enthalten ist; und
- der Härter zu 4-10%, vorzugsweise zu 5-9%, insbesondere bevorzugt zu 6-8% im Kontrastmittel enthalten ist,
- wobei vorzugsweise das Volumen-Verhältnis vom Polyurethan zum Härter im Bereich von 100:10-100:25 liegt, insbesondere bevorzugt im Bereich von 100:16-100:19.

10. Kit-of-Parts für ex vivo Mikroangiografie, aufweisend:
- einen ersten Behälter, der ein iodiertes, verestertes Öl, vorzugsweise ein iodiertes, verestertes Leinsamenöl, insbesondere bevorzugt Ethyl-9, 12, 15-triiodo-octadecatrienoat, enthält, und ein Keton, vorzugsweise 2-Butanon enthält;
- einen zweiten Behälter, der ein Polyurethan, vorzugsweise ein Polyisocyanat-Präpolymer, insbesondere bevorzugt 4,4'-Methylendi(cyclohexyl-isocyanat) enthält; und
- einen dritten Behälter, der einen Härter, vorzugsweise ein modifiziertes aromatisches Diamin, insbesondere bevorzugt 2,6-diamino-3,5-diethyltoluen enthält.

11. Kit-of-Parts gemäss Anspruch 10, wobei der erste Behälter zusätzlich einen Farbstoff, vorzugsweise einen blauen Farbstoff, enthält.

12. Kit-of-Parts gemäss einem der Ansprüche 10-11, wobei
- der erste Behälter eine erste Mischung aus 2-4 ml, vorzugsweise 2.5-2.8 ml des iodierten, veresterten Öls und 2-3 ml, vorzugsweise 2.2-2.9 ml des Ketons enthält; und wobei
- der zweite Behälter 4-7 ml, vorzugsweise 4.5-5 ml des Polyurethans enthält; und wobei
- der dritte Behälter 0.5-1.5 ml, vorzugsweise 0.8-1.2 ml des Härters enthält.

13. Kit-of-Parts gemäss einem der Ansprüche 10-12, des Weiteren aufweisend
- eine erste Spritze zur Aufnahme des Inhalts des ersten Behälters und des zweiten Behälters, vorzugsweise eine Spritze mit 12 ml Volumen;
- eine zweite Spritze zur Aufnahme des Inhalts des dritten Behälters, vorzugsweise eine Spritze mit 1 ml Volumen;
- einen Mischbehälter zur Vermengung des Inhalts der ersten Spritze und der zweiten Spritze;
- vorzugsweise einen Dispenser zur Steuerung der ersten Spritze und der zweiten Spritze, wobei der Dispenser eine Vorrichtung zur Aufnahme eines jeweils ersten Endes der ersten und der zweiten Spritze aufweist;
- und vorzugsweise ein Adapterelement zur Aufnahme eines jeweils zweiten Endes der ersten und der zweiten Spritze und zur Aufnahme eines ersten Endes des Mischbehälters.

14. Verfahren zur Herstellung eines Kontrastmittels für *ex vivo* Mikroangiografie gemäss einem der Ansprüche 1-9, zur digitalen Bildgebung eines Gefässsystems einer Maus oder einer Ratte mittels eines Micro-CT-Geräts, aufweisend die folgenden Schritte:
f) Bereitstellung einer ersten Mischung von iodiertem, veresterten Öl mit einem Keton in einem ersten Behälter;
g) Bereitstellung eines Polyurethans in einem zweiten Behälter;
h) Bereitstellung eines Härters in einem dritten Behälter;
i) Vermengung und Mischen des Inhalts des ersten Behälters mit dem Inhalt des zweiten Behälters zu einer zweiten Mischung;
j) Vermengung des Inhalts des dritten Behälters mit der zweiten Mischung von Schritt i) in einem Mischelement unmittelbar vor der Einspritzung in das zu untersuchende Gefässsystem; wobei vorzugsweise ein Volumen-Mischverhältnis von 100:16 bis 100:19 des Polyurethans zum Härter verwendet wird.

15. Verfahren für ex vivo Mikroangiografie zur digitalen Bildgebung eines Gefässsystems eines tierischen oder menschlichen Körpers oder Organs, inbesondere einer Maus oder einer Ratte, mittels eines micro-CT-Geräts, aufweisend die folgenden Schritte:
k) Bereitstellung eines Kontrastmittels gemäss einem der vorhergehenden Ansprüche 1-9, vorzugsweise nach einem Verfahren gemäss Anspruch 14;
l) Kanülierung und Ausspülung des zu untersuchenden Tierkörpers, vorzugsweise mit einer Klarlösung, insbesondere mit PBS, wobei vorzugsweise für eine Maus eine Spülmenge von 20-100 ml, und vorzugsweise alternativ für eine Ratte eine Spülmenge von 20-200 ml verwendet wird;
m) Injektion des Kontrastmittels in den Körper bzw. in das Organ, vorzugsweise mit gleichmässiger Flussrate, wobei die Flussrate vorzugsweise maximal 3 ml/min, insbesondere bevorzugt maximal 1.5 ml/min beträgt.

16. Verfahren für ex vivo Mikroangiografie nach Anspruch 15, **dadurch gekennzeichnet, dass** nach der Injektion des Kontrastmittels eine Aushärtung des Kontrastmittels im Tierkörper abgewartet wird, und anschliessend der Tierkörper mittels eines Micro-CT-Geräts gescannt wird.

17. Verfahren für ex vivo Mikroangiografie gemäss Anspruch 15 oder 16, **dadurch gekennzeichnet, dass** die Injektion in Schritt m) manuell, vorzugsweise mittels eines Dispensers erfolgt, oder dass die Injektion in Schritt m) mittels einer Injektionspumpe erfolgt.

18. Verwendung eines Kontrastmittels gemäss einem der Ansprüche 1-9 oder eines Kit of Parts gemäss einem der Ansprüche 10-13 für die postmortale Mikroangiografie, **dadurch gekennzeichnet, dass** das Kontrastmittel in einen menschlichen oder tierischen Körper oder in ein menschliches oder tierisches Organ eingespritzt wird.

## Claims

1. Contrast agent for ex *vivo* microangiography, preferably for the digital imaging of a vascular system of a mouse or of a rat or of other laboratory animals, or of single animal and human organs, by means of a micro-CT-device, comprising
- a polyurethane, and
- a hardener,
**characterized in that** the contrast agent additionally contains
- iodized, esterified oil, and
- a ketone.

2. Contrast agent according to claim 1, **characterized in that** the iodized, esterified oil is an iodized, esterified poppy seed oil, or an iodized, esterified linseed oil, wherein the iodized esterified linseed oil preferably is ethyl-9,12,15-triiodo-octadecatrienoate.

3. Contrast agent according to one of the preceding claims, **characterized in that** the ketone is selected from 2-butanone, acetone, or 3-pentanone, wherein the ketone preferably is 2-butanone.

4. Contrast agent according to one of the preceding claims, **characterized in that** the contrast agent comprises a colorant, wherein the colorant preferably is a blue colorant.

5. Contrast agent according to one of the preceding claims, **characterized in that** the polyurethane is a polyisocyanate-prepolymer, preferably an aliphatic isocyanate, in particular preferably 4,4'-methylenedi(cyclohexyl-isocyanate).

6. Contrast agent according to one of the preceding claims, **characterized in that** the hardener is a modified aromatic diamine, wherein the hardener preferably is a diethylmethylbenzenediamine, in particular preferably a mixture of two isomers of diethylmethylbenzenediamine, most preferably an isomeric mixture of 2,6-diamino-3,5-diethyltoluene and 2,4-diamino-3,6-diethyltoluene in a ratio of 7:3.

7. Contrast agent according to one of the preceding claims, **characterized in that**
- the contrast agent contains 20-60%, preferably 22-45%, in particular preferably 24-30% of the iodized, esterified oil; and
- the contrast agent contains 7-30%, preferably 10-25%, in particular preferably 14-22% of the ketone.

8. Contrast agent according to one of the preceding claims, **characterized in that** in a contrast solution, which comprises the iodized, esterified oil and the ketone, the ratio of the volume of the iodized, esterified oil to the volume of the ketone is 0.75-4, preferably 1-1.5, in particular preferably 1.1-1.3.

9. Contrast agent according to one of the preceding claims, **characterized in that** the contrast agent contains 25-60%, preferably 35-55%, in particular preferably 38-50%, and most preferably 43-47% of the polyurethane; and
- the contrast agent contains 4-10%, preferably 5-9%, in particular preferably 6-8% of the the hardener,
- wherein preferably the volume-ratio of polyurethane to hardener is in the range of 100:10-100:25, in particular preferably in the range of 100:16-100:19.

10. Kit-of-parts for ex *vivo* microangiography, comprising:
- a first container, containing an iodized, esterified oil, preferably an iodized, esterified linseed oil, in particular preferably ethyl-9,12,15-triiodo-octadecatrienoate, and a ketone, preferably 2-butanone;
- a second container, containing a polyurethane, preferably a polyisocyanate-prepolymer, in particular 4,4'-methylenedi(cyclohexyl-isocyanate); and
- a third container, containing a hardener, preferably a modified aromatic diamine, in particular preferably 2,6-diamino-3,5-diethyltoluene.

11. Kit-of-parts according to claim 10, wherein the first container additionally contains a colorant, preferably a blue colorant.

12. Kit-of-parts according to one of claims 10-11, wherein
- the first container contains a first mixture of 2-4 ml, preferably 2.5-2.8 ml of the iodized, esterified oil, and 2-3 ml, preferably 2.2-2.9 ml of the ketone; and wherein
- the second container contains 4-7 ml, preferably 4.5-5 ml of the polyurethane; and wherein
- the third container contains 0.5-1.5 ml, preferably 0.8-1.2 ml of the hardener.

13. Kit-of-parts according to one of the claims 10-12, further comprising:
- a first syringe for receiving the contents of the first container and the second container, preferably a syringe with a volume of 12 ml;
- a second syringe for receiving the contents of the third container, preferably a syringe with a volume of 1 ml;
- a mixing container for mixing the contents of the first syringe and the second syringe;
- preferably a dispenser for controlling the first syringe and the second syringe, wherein the dispenser comprises a device for receiving a corresponding first end of the first and of the second syringe;
- and preferably an adapter element for receiving a corresponding second end of the first and of the second syringe and for receiving a first end of the mixing container.

14. Method for the manufacture of a contrast agent for ex vivo microangiography according to one of the claims 1-9, for the digital imaging of a vascular system of a mouse or of a rat by means of a micro-CT-device, comprising the following steps:
f) providing a first mixture of iodized, esterified oil with a ketone in a first container;
g) providing a polyurethane in a second container;
h) providing a hardener in a third container;
i) blending and mixing the contents of the first container with the contents of the second container to form a second mixture;
j) blending of the contents of the third container with the second mixture of step i) in a mixing element immediately before injection into the vascular system to be examined; wherein preferably a volume-mixing ratio of 100:16 to 100:19 of the polyurethane to the hardener is used.

15. Method for *ex vivo* angiography for the digital imaging of a vascular system of an animal or human body or organ, in particular of a mouse or of a rat, by means of a micro-CT-device, comprising the following steps:
k) providing a contrast agent according one of the preceding claims 1-9, preferably according to a method according to claim 14;
l) cannulization and flushing of the animal body to be examined, preferably with a clear solution, in particular with PBS, wherein preferably for a mouse a flushing amount of 20-100 ml, and preferably alternatively for a rat a flushing amount of 20-200 ml is used;
m) injection of the contrast agent into the body or into the organ, respectively, preferably at a uniform flow rate, wherein the flow rate preferably is at the most 3 ml/min, in particular preferably at the most 1.5 ml/min.

16. Method for *ex vivo* angiography according to claim 15, **characterized in that** after the injection of the contrast agent, a hardening of the contrast agent in the animal body is waited for, and subsequently the animal body is scanned by means of a micro-CT-device.

17. Method for *ex vivo* angiography according to claim 15 or 16, **characterized in that** the injection in step m) is carried out manually, preferably by means of a dispenser, or that the injection in step m) is carried out by means of an injection pump.

18. Use of a contrast agent according to one of claims 1-9 or of a kit of parts according to one of claims 10-13 for the post-mortem microangiography, **characterized in that** the contrast agent is injected into a human or animal body or into a human or animal organ.

## Revendications

1. Produit de contraste pour microangiographie *ex vivo,* de préférence pour l'imagerie numérique d'un système vasculaire d'une souris ou d'un rat ou d'autres animaux de laboratoire, ou d'organes individuels animaux et humains, au moyen d'un appareil micro-CT, comprenant
- un polyuréthane, et
- un durcisseur,
**caractérisé en ce que** le produit de contraste comprend en outre
- une huile estérifiée iodée, et
- une cétone.

2. Produit de contraste selon la revendication 1, **caractérisé en ce que** l'huile estérifiée iodée est une huile de graines de pavot estérifiée iodée ou une huile de graines de lin estérifiée iodée, l'huile de graines de lin estérifiée iodée étant de préférence le 9,12,15-triiodo-octadécatriénoate d'éthyle.

3. Produit de contraste selon l'une des revendications précédentes, **caractérisé en ce que** la cétone est choisie parmi la 2-butanone, l'acétone ou la 3-pentanone, la cétone étant de préférence la 2-butanone.

4. Produit de contraste selon l'une des revendications précédentes, **caractérisé en ce que** le produit de contraste contient un colorant, le colorant étant de préférence un colorant bleu.

5. Produit de contraste selon l'une des revendications précédentes, **caractérisé en ce que** le polyuréthanne est un prépolymère de polyisocyanate, de préférence un isocyanate aliphatique, de manière particulièrement préférée le 4,4'-diisocyanate de dicyclohexylméthylène.

6. Produit de contraste selon l'une des revendications précédentes, **caractérisé en ce que** le durcisseur est une diamine aromatique modifiée, le durcisseur étant de préférence une diéthylméthylbenzènediamine, de manière particulièrement préférée un mélange de deux isomères de diéthylméthylbenzènediamine, de manière préférée entre toutes un mélange d'isomères de 2,6-diamino-3,5-diéthyltoluène et 2,4-diamino-3,6-diéthyltoluène dans un rapport de 7:3.

7. Produit de contraste selon l'une des revendications précédentes, **caractérisé en ce que**
- l'huile estérifiée iodée est contenue dans le produit de contraste à 20-60%, de préférence 22-45%, de manière particulièrement préférée à 24-30%; et
- la cétone est contenue dans le produit de contraste à 7-30%, de préférence à 10-25%, de manière particulièrement préférée à 14-22%.

8. Produit de contraste selon l'une des revendications précédentes, **caractérisé en ce que** dans une solution de contraste contenant l'huile estérifiée iodée et la cétone, le rapport du volume de l'huile estérifiée iodée au volume de la cétone est de 0,75-4, de préférence 1-1,5, en particulier 1,1-1,3.

9. Produit de contraste selon l'une des revendications précédentes, **caractérisé en ce que** le polyuréthane est contenu dans le produit de contraste à 25-60%, de préférence 35-55%, de manière particulièrement préférée 38-50%, et de préférence entre toutes à 43-47%; et
- le durcisseur est contenu dans le produit de contraste à 4-10%, de préférence à 5-9%, de manière particulièrement préférée 6-8%,
- le rapport volumique du polyuréthane au durcisseur étant de préférence dans la plage de 100:10 - 100:25, de manière particulièrement préférée dans la plage de 100:16 - 100:19.

10. Kit de pièces pour microangiographie *ex vivo*, comprenant:
- un premier récipient qui contient une huile estérifiée iodée, de préférence une huile de graines de lin estérifiée iodée, de manière particulièrement préférée le 9,12,15-triiodo-octadécatriénoate d'éthyle, et une cétone, de préférence la 2-butanone;
- un deuxième récipient, qui contient un polyuréthane, de préférence un prépolymère de polyisocyanate, de manière particulièrement préférée, le 4,4'-diisocyanate de dicyclohexylméthylène; et
- un troisième récipient, qui contient un durcisseur, de préférence une diamine aromatique modifiée, de manière particulièrement préférée le 2,6-diamino-3,5-diéthyltoluène.

11. Kit de pièces selon la revendication 10, dans lequel le premier récipient contient en outre un colorant, de préférence un colorant bleu.

12. Kit de pièces selon l'une des revendications 10 - 11, dans lequel
- le premier récipient contient un premier mélange de 2 - 4 ml, de préférence 2,5 - 2,8 ml d'huile estérifiée iodée et 2 - 3 ml, de préférence 2,2 - 2,9 ml de cétone; et où
- le deuxième récipient contient 4 - 7 ml, de préférence 4,5 - 5 ml de polyuréthane; et où
- le troisième récipient contient 0,5 à 1,5 ml, de préférence 0,8 à 1,2 ml de durcisseur.

13. Kit de pièces selon l'une des revendications 10 - 12, comprenant en outre
- une première seringue pour recevoir le contenu du premier récipient et du deuxième récipient, de préférence une seringue d'un volume de 12 ml;
- une deuxième seringue pour recevoir le contenu du troisième récipient, de préférence une seringue d'un volume de 1 ml;
- un récipient de mélange pour mélanger le contenu de la première seringue et de la seconde seringue;
- de préférence un distributeur pour commander la première seringue et la deuxième seringue, le distributeur comportant un dispositif pour recevoir une première extrémité de la première et de la deuxième seringue, respectivement;
- et de préférence un élément adaptateur pour recevoir une deuxième extrémité de la première et de la deuxième seringue, respectivement, et pour recevoir une première extrémité du récipient de mélange.

14. Procédé de production d'un produit de contraste pour microangiographie *ex vivo* selon l'une des revendications 1 à 9, pour l'imagerie numérique d'un système vasculaire d'une souris ou d'un rat au moyen d'un appareil micro-CT, comprenant les étapes suivantes:
f) fournir un premier mélange d'huile estérifiée iodée avec une cétone dans un premier récipient;
g) fournir un polyuréthane dans un deuxième récipient;
h) fournir un durcisseur dans un troisième récipient;
i) combiner et mélanger le contenu du premier récipient avec le contenu du deuxième récipient pour former un deuxième mélange;
j) combiner le contenu du troisième récipient avec le deuxième mélange de l'étape i) dans un élément de mélange immédiatement avant l'injection dans le système vasculaire à examiner; de préférence en utilisant un rapport de mélange en volume de 100:16 à 100:19 de polyuréthanne et durcisseur.

15. Procédé de microangiographie *ex vivo* pour l'imagerie numérique d'un système vasculaire d'un corps ou d'un organe animal ou humain, en particulier d'une souris ou d'un rat, au moyen d'un appareil micro-CT, comprenant les étapes suivantes:
k) fournir un produit de contraste selon l'une des revendications 1 à 9 précédentes, de préférence selon un procédé selon la revendication 14;
l) canulation et rinçage du corps animal à examiner, de préférence avec une solution claire, en particulier avec du PBS, une quantité de rinçage de 20-100 ml étant utilisée de préférence pour une souris, et de préférence une quantité de rinçage de 20-200 ml pour un rat;
m) injection du produit de contraste dans le corps ou dans l'organe, de préférence à débit constant, le débit étant de préférence au maximum de 3 ml/min, de manière particulièrement préférée au maximum de 1,5 ml/min.

16. Procédé de microangiographie *ex vivo* selon la revendication 15, **caractérisé en ce que**, après l'injection du produit de contraste, le durcissement du produit de contraste dans le corps de l'animal est attendu et que le corps de l'animal est ensuite scanné au moyen d'un appareil micro-CT.

17. Procédé de microangiographie *ex vivo* selon la revendication 15 ou 16, **caractérisé en ce que** l'injection à l'étape m) a lieu manuellement, de préférence au moyen d'un distributeur, ou **en ce que** l'injection à l'étape m) a lieu au moyen d'une pompe d'injection.

18. Utilisation d'un produit de contraste selon l'une quelconque des revendications 1 - 9 ou d'un kit de pièces selon l'une des revendications 10 à 13 pour la microangiographie *post mortem*, **caractérisée en ce que** le produit de contraste est injecté dans un corps humain ou animal ou dans un organe humain ou animal.
